# EUROPEAN PATENT APPLICATION

(11) **EP 1 617 348 A1**
(43) Date of publication of application: **18.01.2006**
(21) Application number: 05254325.3
(22) Date of filing: 11.07.2005
(51) Int. Cl.: G06F 19/00

(54) **Method for recommending an acne treatment/prevention program**

(30) Priority: 12.07.2004 US 889496
(71) Applicant: JOHNSON & JOHNSON CONSUMER COMPANIES, INC., Skillman, NJ 08558 (US)
(72) Inventor: Wiegand, Benjamin, Yardley, PA 19067 (US); Luedtke, Kathryn, Doylestown, PA 18901 (US); Rapp, Stephen Robert, Winston-Salem, NC 27104 (US)
(74) Representative: Mercer, Christopher Paul

(57) **Abstract**

The invention relates to an acne profile of an individual comprising an acne quality of life score which is determined by obtaining and analyzing acne quality of life information. The profile may also optionally contain an acne severity score which is determined by obtaining and analyzing acne Severity information and/or a stress score which is determined by obtaining and analyzing stress information from the individual.

## Description

### 1. FIELD OF THE INVENTION

The invention relates to an acne profile for use in assessing the acne condition of an individual. The acne profile is an improvement over previous means of assessing acne in that it considers the impact of acne on the individual's quality of life. The acne profile comprises an acne quality of life score which is determined by obtaining and analyzing acne quality of life information from the individual. In another embodiment, the invention relates to a method of recommending an acne treatment or prevention program to an individual. The method comprises profiling the acne condition of the individual using the acne profile discussed above and then recommending an acne treatment or prevention program.

### 2. BACKGROUND AND DESCRIPTION OF THE PRIOR ART

Acne vulgaris is a skin condition that affects over 85% of adolescents and young adults. The following are four primary factors that lead to the formation of acne vulgaris; (1) increased sebum output resulting in oily, greasy skin; (2) increased bacterial activity normally due to an overabundance of *Propionibacterium acnes;* (3) plugging (hypercornification) of the follicle or pilosebaceous duct; and (4) production of inflammation by substances leaking into the dermis which cause inflammatory reactions. The major physical ramification of acne is the appearance of lesions on the face, chest and/or back areas. Acne lesions change over time from blackheads and whiteheads to inflammatory lesions (papules and pustules) that upon healing may leave pigmentary changes, cysts, or scars. A more detailed description can be found in Cunliffe, William J., Acne, London: Martin Duitz Ltd., 1989, Chapter 1, the disclosure of which is hereby incorporated by reference.

Numerous methods are available for measuring the severity of an individual's acne condition. The most widely used method is a dermatological evaluation using an established acne severity scale. Examples of known acne severity scales include the Cook scale, Leed's Acne Severity Grading Technique, and Allen's Acne Severity Index. See, for example, Cook CH, Centner RL, Michaels SE. An acne grading method using photographic standards. Arch Dermatol 1979; 115: 571-575.; Burke BM, Cunliffe WJ, The assessment of acne vulgaris - the Leeds technique, Br J Dermatol 1984; 111: 83-92.; and Allen BB, Smith JG. Various parameters for grading acne vulgaris. Arch Dermatol 1982; 118: 23-25, the disclosures of which are hereby incorporated by reference. More recently, advanced imaging and instrumental methods have been developed for measuring acne severity. Examples include the use of imaging analysis such as taught by U.S. published patent application Nos. 2003/00867112; 2003/0086703; 2003/0138249; and 2002/0161664, the disclosures of which are hereby incorporated by reference.

While the physical symptoms of acne are no doubt a primary concern for an acne patient, the psychological effects of acne can be quite significant. Unfortunately, however, the psychological effects of acne are quite commonly overlooked in assessing an individual's acne condition. As stated by Sulzberger and Zaidens in Sulzberger MB, Zaidens, SH. Psychogenic factors in dermatologic disorders. Med Clin North Am 1948; 32: 669-685. and incorporated here by reference, "There is no single disease which causes more psychic trauma...than does acne vulgaris." In particular, acne is believed to have a significant negative impact on an individual's "skin related quality of life", i.e., the functional, emotional and social effects of diseases that affect one's skin, for example, the way an individual's skin makes them feel or the impact of his skin on the individual's self-esteem. A more detailed description of skin-related quality of life is available in Rajagopaln, RR; Sherertz, ER; Anderson, RT. Care Management of Skin Diseases: Life Quality and Economic Impact. New York: Marcel Dekker, Inc. 1998, Chapter 4, the disclosure of which is hereby incorporated by reference.

Although the problem is clear, the assessment tools available for assessing the impact of acne on skin-related quality of life are limited. Examples of methods for assessing the impact of general skin condition on quality of life can be found in the following journal articles, the disclosures of which are hereby incorporated by reference: Jowett S, Ryan T. Skin disease and handicap: an analysis of the impact of skin conditions. Social Science and Medicine 1985; 20; 425-429.; Krowchuk Dp, Stancin T, Keskinen R, Walker R, Bass J, Anglin TM. The psychosocial effects of acne in adolescents. Pediatric Dermatology 1991; 4(332): 338.; Lewis-Jones MS, Finlay AY. The Children's Dermatology Life Quality Index (CDLQU): an initial validation and practical use. Br J Dermatol 1995; 132(6): 942-949.; Motley RY, Finlay AY. How much disability is caused by acne? Clinical and Experimental Dermatology 1989; 14: 194-198.; Wu SF, Kinder BN, Trunnell TN, Fulton JE. Role of Anxiety And Anger In Acne Patients: A Relationship With The Severity Of The Disorder Journal of the American Academy of Dermatology 1988; 18: 325-333. Because other skin conditions may affect quality of life differently than acne, these general skin condition-related measures are limited in use for acne because they are not sensitive to aspects of quality of life affected specifically by acne.

While specific to acne, existing acne-related health-related quality of life measures such as the Acne Disability Index (Motley RJ, Finlay AY. Clin Exper Dermatol 1989; 14: 194-198.), the Acne Quality of Life Scale (Gupta MA, Johnson AM, Gupta Ak. Acta Dermatologica Venereological 1998; 78(6): 451-456.), the Acne Specific Quality of Life Questionnaire (Giman, C.J. et al. Quality of Life Research 1996; 5: 481-490.) and the Acne QOL Questionnaire (Martin, A.R. et al. Clinical and Experimental Dermatology 2001; 26: 380-385.) are limited in the sense that they tend to confound direct severity variables with quality of life variables. This is a major shortcoming because the impact of an individual's acne condition on his quality of life is not necessarily correlated to the severity of the individual's acne.

An example of information confounded with severity is the item "My skin bleeds", found in the Skindex, a skin-related quality of life measure. Further details on the Skindex can be found in Chren, M. "Improved Discriminative and Evaluative Capability a Refined Version of Skindex, a Quality of Life Instrument for Patients with Skin Disease," Arch Dermatolo. 1997; 133: 1443-1440, the disclosure of which is hereby incorporated by reference. While this item is somewhat reflective of the degree to which the individual perceives their acne as affecting their quality of life, it also depends on the severity of the individual's acne. Since the Skindex final scores include the individual's response to severity-like items such as this, it is difficult to determine how much of the score is due to acne severity versus the individual's perception of acne's impact on their quality of life.

This is a major shortcoming because, as the present inventors have discovered, the impact of an individual's acne condition on his quality of life is not necessarily correlated to the severity of the individual's acne. For example, an individual with low acne severity may nonetheless experience highly diminished acne quality of life. Such an individual would clearly benefit from a treatment program that addresses acne quality of life issues. However, if the assessment of his acne condition only included acne-severity information, the negative impact on the individuals quality of life would be left untreated. Accordingly, there remains a need for a method for assessing the impact of an individual's acne condition on the individual's quality of life which is not confounded with severity. The present invention answers this need.

### 3. SUMMARY OF THE INVENTION

The invention relates to an acne profile of an individual comprising an acne quality of life score which is determined by obtaining and analyzing acne quality of life information. The profile may also optionally contain an acne severity score which is determined by obtaining and analyzing acne severity information and/or a stress score which is determined by obtaining and analyzing stress information from the individual.

In another embodiment, the invention relates to a method of recommending an acne treatment or prevention program to an individual. The method comprises profiling the acne condition of the individual using the acne profile discussed above and then recommending an acne treatment or prevention program.

The invention also relates to a method of assessing the efficacy of an acne treatment or prevention program. The method comprises profiling the baseline acne condition of an individual ("profiled baseline acne condition") prior to the administration of said acne treatment or prevention program; profiling the acne condition of an individual after administration of an acne treatment or prevention program ("profiled after-treatment acne condition"); and then comparing the profiled baseline acne condition to the profiled after-treatment acne condition to determine the efficacy of said acne treatment or prevention program. The profiling step uses the acne profile discussed above.

In yet another embodiment, the invention relates to a method of selecting an acne treatment or prevention program, said method comprising:
a. assessing the efficacy of two or more proposed acne treatment or prevention programs as discussed above;
b. comparing the efficacy of the two or more proposed acne treatment or prevention programs; and
c. selecting the most efficacious acne treatment or prevention program.

### 4. DETAILED DESCRIPTION OF THE INVENTION

As discussed above, the inventors have discovered an acne profile for use in assessing the acne condition of an individual which is an improvement over previous means in that it considers the impact of acne on the individual's quality of life and is not confounded with severity. The acne profile comprises an acne quality of life score which is determined by obtaining and analyzing acne quality of life information from the individual.

### A. Acne Quality Of Life Information

As discussed above, the acne profile according to the invention comprises an acne quality of life score which is determined by obtaining and analyzing acne quality of life information from the individual being profiled. As used herein "acne quality of life" means the impact of one's acne condition on his quality of life. As discussed above, the inventors have discovered that this impact may or may not be correlated to the severity of the individual's acne. For instance, an individual with low acne severity may nonetheless experience highly diminished quality of life as a result of their acne condition. For this reason, acne quality of life information must not be confounded with acne severity information. Thus, acne quality of life information must be collected distinct from acne severity information. By this definition, quality of life information that includes information on the severity of an individual's acne condition would not be considered true "acne quality of life" information.

In one embodiment of the invention, acne quality of life information is collected on one or more dimensions of acne quality of life. Acne quality of life dimensions include the impact of an individual's acne on social functioning, psychological functioning, including self-esteem and/or interpersonal sensitivity, mood, relationship functioning, and the burden of complying with current or proposed acne treatment program(s) and the satisfaction with previously or currently used acne treatment programs. Other dimensions that relate to acne's impact on an individual's lifestyle, health, well-being and emotional state would also be particularly useful and are deemed to be within the scope of this invention.

There are many different specific pieces of information that are useful to collect within each dimension. In one embodiment, acne quality of life information may comprise the answers to questions that pertain to the impact of an individual's acne condition on his quality of life. For example, it is useful to gather information on the impact of acne on the individual's mood by asking the degree to which the individual's acne condition has impacted how happy, angry, sad, uneasy, frustrated, mad, uncomfortable, ashamed, stressed out, anxious, nervous, down on himself, touchy or upset he feels. It is also useful to gather information on the impact of acne on the individual's psychological functioning by asking the degree to which the individual's acne condition has made him feel unhappy with his appearance, less self-confident, discouraged, humiliated, hopeless about his skin, ugly, self-pitying, alone or like he can't be himself. It is also useful to gather information on the impact of acne on the individual's social functioning by asking the degree to which the individual's acne condition has made him feel uncomfortable around others, self-conscious around others, like he wants to avoid other people, embarrassed, unattractive to others, stared at, or afraid of encountering people. It is also useful to gather information on the impact of acne on the individual's relationships by asking the individual how concerned he has been about being around friends and family, being around strangers, being around members of the opposite sex (same sex if gay), what other's think of him, concealing his acne from others, being unattractive to others, being in public places, not letting others see his blemishes, avoiding social gatherings, not letting others touch him, his love relationships, his future, not being accepted by others, or getting physically close to others. It is also useful to gather information on the satisfaction of the individual with regards to his acne treatment by asking how satisfied he has been with how well his acne treatment or products work, the cost of his current acne treatment or products, how easy it is to use the acne treatment or product, or the time he spends taking care of his acne. It may also be useful to gather information on any positive impacts of acne on the individual by asking, in regards to his acne, how much the individual has felt peaceful, at ease, confident, patient, hopeful, calm, in control, compassionate toward others, friendly toward himself, comfortable, normal, pretty or handsome, accepting of life and himself, carefree, accepted by others, friendly toward others, attractive to others, connected to others, outgoing, with his mind at ease, willing to approach people, or like 'going with the flow' of life.

In one embodiment, the acne quality of life information is collected using a psychometric tool. As used herein, a "psychometric tool" refers to a psychological questionnaire which has been tested to be valid and reliable by known methods of validating and testing reliability.

In a preferred embodiment, the psychometric tool used to collect acne quality of life information is the Acne Quality of Life Index ("AQOLI"). The AQOLI is a 21-item index that assesses the impact of acne on three dimensions: social functioning, psychological functioning, and mood. The AQOLI is superior to previously developed assessment tools as it does not confound the score with acne severity and it has been tested and shown to have excellent validity and reliability. The AQOLI items and format is shown in Table 1. The AQOLI is scored by summing the individual's numerical responses, resulting in a minimum sum of 21 and a maximum sum of 147. This sum is then subtracted from the maximum 147 to yield an AQOLI score. The final AQOLI scores thus range from 0 (147-147 = 0), representative of the lowest measureable acne quality of life, to 126 (147-21 = 126), representative of the highest measurable acne quality of life. A higher score on the AQOLI represents a more negative quality of life, i.e. indicates a greater negative impact of acne on the individual's quality of life. A lower score on the AQOLI represents a less negative impact of acne on the individual's quality of life. Thus, a treatment designed to decrease acne quality of life would be deemed successful if it decreased an individual's AQOLI score. Other psychometric tools that similarly assess dimensions of acne quality of life would also be particularly useful and are deemed to be within the scope of this invention.

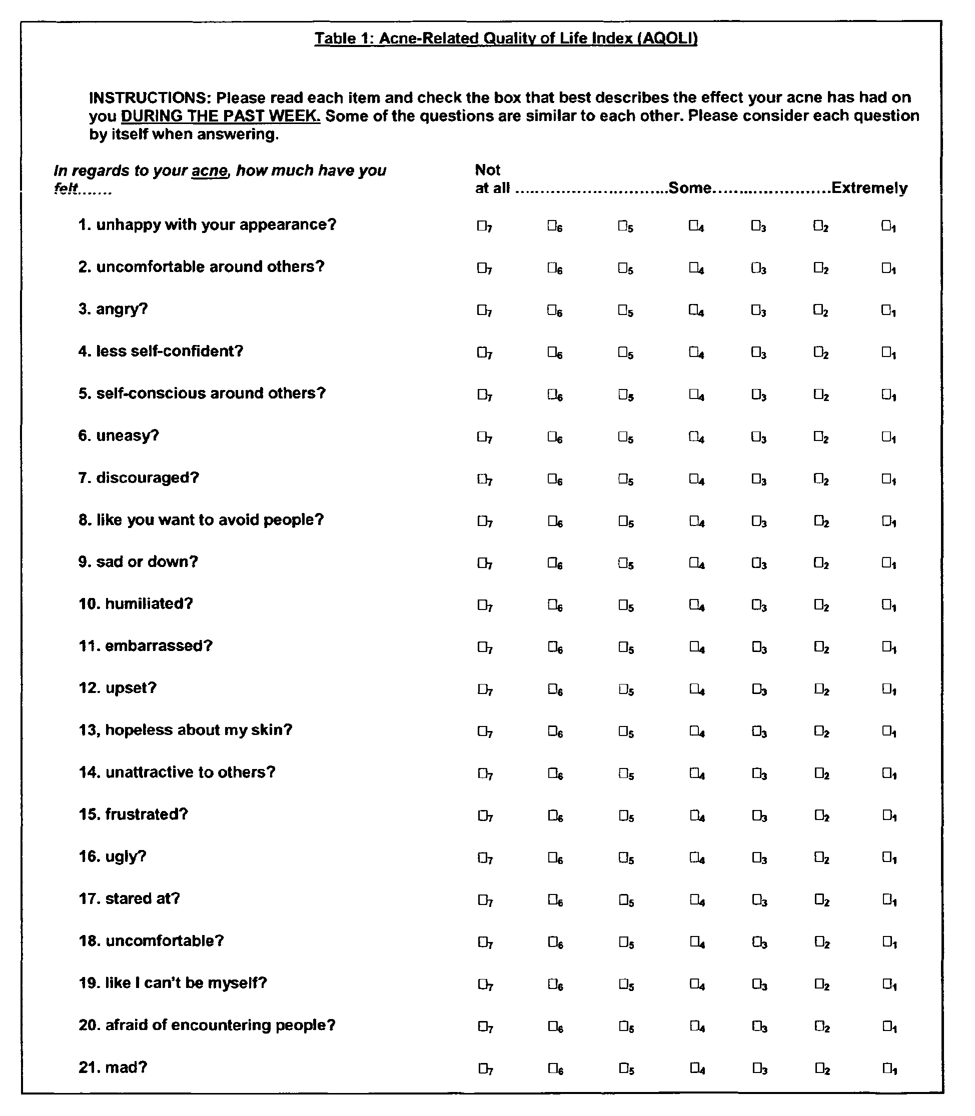

### B. Acne Severity Information

Optionally, the profile according to the invention further comprises information relating to acne severity, i.e. an acne severity score which is determined by obtaining and analyzing acne severity information from the individual being profiled.

As used herein, "acne severity" refers specifically to the physical symptoms of acne, i.e., the localized physical impact of acne on the skin, including for example, visible symptoms such as pimples, redness, lesions, etc. and non- visible symptoms such as stinging, soreness, itching, pressure, etc. of the individual. Any method used to gather such information from an individual may be used. In one embodiment, acne severity information comprises at least one of the following: number of pimples, severity of the pimples, degree of dryness, degree of oiliness, degree of redness, number of scars, degree of skin roughness, amount of skin pus, number of blackheads, number of whiteheads, degree of pigmentation, and combinations thereof. Other physical skin symptoms of acne are also deemed to be included in acne severity information.

Numerous methods are available for collecting an individual's acne severity information. A widely used method is a dermatological evaluation or grading using one of a number of established acne severity scale such as the Cook scale, Leed's Acne Severity Grading Technique and Allen's Acne Severity Index as described in Cook CH, Centner RL, Michaels SE. An acne grading method using photographic standards. Arch Dermatol 1979; 115: 571-575.; Burke BM, Cunliffe WJ, The assessment of acne vulgaris - the Leeds technique, Br J Dermatol 1984; 111: 83-92.; and Allen BB, Smith JG. Various parameters for grading acne vulgaris. Arch Dermatol 1982; 118: 23-25, the disclosures of which are hereby incorporated by reference. Self-report scales may also be used. A self-report scale can be as simple as asking an individual to keep track of the number of pimples that they observe over a period of time or as complicated as an elaborate multi-dimensional scale on which an individual records numerous attributes of the individual's skin and their changes over a period of time. Few self-report scales exist in the literature as they tend to be custom made depending on the specific acne information one cares about.

More recently, advanced imaging and instrumental methods have been developed for measuring acne severity. For example, instruments are available to measure the amount of sebum secreted by an individual's skin or the amount of bacteria present on an individual's skin. For example, U.S. published patent application No. 2003/0086703 discloses a method to visually assess the presence of bacteria via imaging. Other methods to assess acne severity include the use of imaging analysis such as taught, for example, by U.S. published patent application Nos. 2003/00867112; 2003/0086703; 2003/0138249; and 2002/0161664, the disclosures of which are hereby incorporated by reference.

Acne severity information can be collected by visual assessment of the individual. This visual assessment can either be done by the individual himself or by someone else. The person performing the visual assessment can either be trained or untrained. Visual assessment can either be done by looking at the individual's skin or by looking at an image of the individual's skin. In doing a visual assessment, the person doing the assessment may record qualitative information like whether or not the person's acne condition appears to be severe or mild. This information may or may not be associated with a quantitative scale such as one from 1 to 5, where 1 = mild and 5 = severe.

### C. Stress Information

A preferred use of our invention involves the inclusion of stress information in the acne profile. Stress, which is present among both genders and all age groups, but clearly present among the teen-age and 20 -30 year old populations, can exacerbate and lead to acne problems via a number of different pathways. For example, chronic stress may lead to a reduction in the overall immune system, which may affect the skin's ability to fight off the *Propionibacterium acnes* bacteria. Accordingly, the profile according to the invention optionally further comprises information relating to stress, i.e. a stress score which is determined by obtaining and analyzing stress information from the individual being profiled.

As used herein, "stress" refers to either acute stress or chronic stress where chronic stress refers to the built up effects of stressors over an extended period of time and acute stress refers the amount or impact of stressors occurring in the immediate or recent time period. Stress information can be collected either psychologically or physiologically.

Stress information can be collected psychologically using surveys, questionnaires, validated psychometric tools or any other method providing information relating to a person's degree of stress. For example, a questionnaire that simply asks an individual to record how much stress he experiences is one suitable method of collecting stress information. In a preferred embodiment, stress information is collected using a stress psychometric tool. A detailed description of many suitable stress psychometric tools can be found in Cohen S, Kessler K and Underwood Gordon L. Measuring Stress: A guide for Health & Social Scientists, New York: Oxford University Press, 1997, Chapters 4-7, the disclosure of which is incorporated herein by reference. Examples of suitable stress psychometrics include the the Trier Inventory of Chronic Stress, version 1 (Schulz, P. and Schlotz, W. Diagnostica, 1999; 45: 8-19.); the Trier Inventory of Chronic Stress, Version 2 (TICS 2), Schulz, P., & Schlotz, W. (2002) [Das Trierer Inventar zur Erfassung von chronischem Stress - Version 2 (TICS 2)], Trierer Psychologische Berichte, Band 29, Heft 2. Trier: Universität, Fachbereich I - Psychologie); the Perceived Stress Scale (Cohen, S. Kamarck, T. and Mermelstein, R. A global measure of perceived stress. Journal of Health & Social Behavior, 1983; 24: 385-396.); and the State-Trait Anxiety Inventory (Stait-Trait Anxiety Inventory: Spielberger, CD. and Vagg, PR. J. Pers. Assess. 1984, Feb.; 48(1): 95-97), the disclosures of which are hereby incorporated by reference.

In a preferred embodiment, stress information is collected using the Trier Inventory of Chronic Stress, version 2, (TICS-2). The TICS-2 is a 62-item questionnaire that assesses various dimensions of an individual's chronic stress. The eleven dimensions in the TICS-2 are Work overload, Performance pressure at work, Social responsibility, Performance pressure in social situations, Overextended at work, Social isolation, Social failure, Worry propensity, Aversive work load, Fatigue, and Social conflicts. The TICS-2 is particularly useful in collecting stress information because it shows the relative importance of multiple dimensions of stress in the individual's life. Other psychometric tools that similarly assess multiple dimensions of stress would also be particularly useful and are deemed to be within the scope of this invention.

Additionally, there are a number of physiological ways to collect stress information. Physiological means of gathering stress information include biometric tools such as skin conductance, heart rate, heart rate variability, blood pressure, skin temperature or assessment of stress-related biomarkers in blood or saliva. Diffuse Reflectance Spectroscopy (DRS), as described in copending U.S. Paten Application No. 10/353,525 the disclosure of which is hereby incorporated by reference, is another physiological way of collecting stress information. Chronic stress, the accumulated stress over time, is most important in influencing an individual's acne condition. Thus, a preferred method of measuring stress is the objective, non-invasive method of using salivary cortisol levels described in detail in copending U.S. Patent Application Nos. 10/012,627 filed December 7, 2001 and U.S. Patent Application No. 10/017,180, the disclosures of which are hereby incorporated by reference.

### D. Assessing Acne Conditions

In one embodiment of the invention, the acne profile according to the invention is used to assess the acne condition of an individual. For example, a baseline acne profile may be compared to a benchmark acne profile. The baseline acne profile may be an acne profile of an individual taken at a time point of interest such as before administration of a treatment program. The benchmark acne profile could consist of a pre-established data set of acne profiles or an acne profile collected from the same individual at an earlier timepoint.

Comparison of an individual's baseline acne profile to a pre-established data set of acne profiles is of particular value because it provides information about the degree of an individual's acne condition relative to other populations of interest. This could help in determining acne treatment or prevention regimens as the individual could be given a treatment or prevention regimen shown to be effective on people with similar acne profiles.

Comparing the acne profile of an individual to a benchmark consisting of the individual's acne profile at a previous timepoint is useful because it could show what effect a treatment or prevention program administered within the given time period had on the individual's acne.

Examples of benchmark data sets include a collection of acne profiles from people of the same age and gender of the individual, a collection of acne profiles from people with the same degree of acne severity or a collection of acne profiles of the individual at different points in time such as different times of year or at different stages of acne treatment. The benchmark data set could be contained in a spreadsheet or an intemet database. Preferably, statistical information such as mean, median, and trends would be available for the benchmark data set.

Comparing the individual's acne profile to the benchmark data set could include plotting the individual's acne profile data on a graph versus the benchmark data or calculating the difference between the individual's acne profile scores versus the mean or median scores from the benchmark data set. Other graphical, mathematical, and statistical analyses of the individual's acne profile versus the benchmark data set are possible and are deemed within the scope of this invention.

### E. Recommending An Acne Treatment or Prevention Proqram

In another embodiment, the invention relates to a method of recommending an acne treatment or prevention program to an individual. The method comprises profiling the acne condition of the individual and then recommending an acne treatment or prevention program. The profiling step comprises determining an acne quality of life score by obtaining and analyzing acne quality of life information from the individual as discussed above. Optionally, the profiling step may also include determining an acne severity score by obtaining and analyzing acne severity information as previously discussed and/or a stress score determined by obtaining and analyzing stress information as previously discussed.

The recommended acne treatment or prevention program may comprise products, services, activities, education, counseling or a combination thereof. Any method useful for improving the lifestyle, health, well-being and/or emotional state of an individual would be useful as an element that addresses acne quality of life issues and is deemed to be within the scope of this invention. For example, the acne treatment or prevention program may include a recommendation to seek psychological, emotional, and/or social counseling, start an exercise program, including for example, running, sports, tai chi, yoga or pilates, practice behavioral modification techniques, relaxation techniques and/or breathing techniques, improve sleep patterns, modify current acne treatment and/or prevention program so that it is less burdensome or more satisfying to the individual; and any other activity that would improve the individual's lifestyle, health, well-being and emotional state, including reading a book or magazine, watching movies, taking a vacation, meditation. Other examples include the administration of herbal supplements, vitamins or special food, and/or over the counter or prescription medications.

In one embodiment the invention relates to a method of providing an acne treatment or prevention program as discussed above, wherein if the acne quality of life score for said individual is higher than a benchmark acne quality of life score, than an acne treatment or prevention program that includes elements that address acne quality of life is recommended. Further, if the acne severity score for said individual is higher than a benchmark acne severity score, then an acne treatment or prevention program that includes elements that address acne severity is recommended.

Methods useful for treating acne quality of life issues may also be used to treat stress issues. Any method that is useful for treating stress can be used as an element for addressing stress issues in the present invention. For example, practicing a sensory regimen as described in copending U.S. Patent Application No. 10/012627, the disclosure of which is hereby incorporated hy reference.

In another embodiment, the recommended acne treatment or prevention program may comprise elements that address acne severity, i.e., elements that would decrease the individual's acne severity. For example, over the counter commercially available anti-acne agents for topical use may be recommended. These include, for example, salicylic acid, sulfur, lactic acid, glycolic acid, pyruvic acid, urea, resorcinol, N-acetylcysteine, retinoic acid, benzoyl peroxide, octopirox, triclosan, azelaic acid, phenoxyethanol, phenoxypropanol, flavinoids, derivatives thereof, and combinations thereof. In addition to the products that are available over the counter, there are also a number of different pharmaceutical treatments that have been developed for the treatment of acne. These include, but are not limited to both topical and oral agents, for example isotretinoin and tretinoin, adapalene, tazarotene, azelaic acid, minocycline, doxycycline, antibacterials such as erythromyacin and clindamycin, vitamins such as zinc, folic acid and nicotinamide, as well as combinations of these antibacterials with over the counter anti-acne agents. Any method useful for treating acne conditions would be useful and are deemed to be within the scope of this invention.

The invention also relates to a method of assessing the efficacy of an acne treatment or prevention program. The method comprises profiling the baseline acne condition of an individual ("profiled baseline acne condition") prior to the administration of said acne treatment or prevention program; profiling the acne condition of an individual after administration of an acne treatment or prevention program ("profiled after-treatment acne condition"); and then comparing the profiled baseline acne condition to the profiled after-treatment acne condition to determine the efficacy of said acne treatment or prevention program. The profiling step uses the acne profile discussed above optionally using acne severity and/or stress information.

In yet another embodiment, the invention relates to a method of selecting an acne treatment or prevention program to be used by an individual affected by acne. The method comprises:
a. assessing the efficacy of two or more proposed acne treatment or prevention programs as discussed above;
b. comparing the efficacy of the two or more proposed acne treatment or prevention programs; and
c. selecting the most efficacious acne treatment or prevention program for the individual's use.

Several examples are set forth below to further illustrate the nature of the invention and the manner of carrying it out. However, the invention should not be considered as being limited to the details thereof.

### 5. EXAMPLES

### Example A. Subsets established by an acne quality of life measure (AQOLI) + an acne severity measure (self-report data)

A group of 480 panelists with self-perceived acne participated in a one-time study to determine their acne profile. To assess acne severity, the panelists completed a self-report acne severity scale. The scale consisted of eight, self-rated items describing current facial acne severity assessing oiliness (1 = "Not at all" to 7 = "Extremely oily"), blackheads (1 = "None" to 7 = "Extremely numerous"), small red bumps (1 ="None" to 7 = "More than 10"), large red bumps (1 = "None" to 7 = "More than 10"), pus bumps (1 = "None" to 7 = "More than 10"), scabs on face, (1 = "None" to 7 = "More than 10"), area of discoloration on face (1 = "None" to 7 = "More than 10 dimes"), and area of the face affected by acne (1 = "None" to 7 = "Extensive area"). A total severity score was computed by summing items with total scores falling between 8 and 56. Internal consistency of the severity items was high (Cronbach's alpha = 0.81).

To assess their acne quality of life, the panelists completed the Acne Quality of Life Index (AQOLI). A higher score on the AQOLI represents a more negative quality of life, i.e. indicates a greater negative impact of acne on the individual's quality of life, and a lower score on the AQOLI represents a less negative impact of acne on the individual's quality of life.

An acne quality of life score and an acne severity score were calculated for each individual. In order to establish subsets of the panelist population, each individual's scores were classified as high (greater than the mean) or low (less than the mean). In order to understand how severity relates to acne quality of life, a count was taken to determine how many individuals had high vs. low, severity vs. acne quality of life. A summary of this quantification is presented in Table A1.

**Table A1: Individual Acne Severity Score vs. AQOLI Score Classification Combinations**

| **Group** | **Level of Acne Severity (Severity Score)** | **Level of Acne Quality of Life (AQOLI Score)** | **Number of Participants** |
|---|---|---|---|
| 1 | High | High | 134 |
| 2 | High | Low | 70 |
| 3 | Low | High | 73 |
| 4 | Low | Low | 204 |

As seen in Table A1, there are individuals in each possible subgroup. This is surprising because the common belief is that the level of acne impact on quality of life would be directly related to the severity of acne. Groups 2 and 3, respectively, illustrate that there are individuals who have high acne severity but low impact on quality of life as well as individuals who have low acne severity by high impact on quality of life. Without the use of quality of life measures, the differences in the acne population would not be clear. Further, the use of a combination of acne quality of life and acne severity scores allows one to select a treatment customized to the individual's needs in terms of both severity and quality of life. Current dermatological practice utilizes only severity as its measure of a population's acne. As shown here, the two populations identified by severity level greatly differ in terms of the impact that acne is having on their quality of life. Thus, treatment based entirely on severity may not be as effective as it does not address the differences in acne's impact on quality of life.

### Example B. Subsets established by an acne quality of life measure (AQOLI) + an acne severity measure (self-report data) + a stress measure (PSS)

This example illustrates the utility of an acne profile that combines both acne quality of life information and stress information with acne severity information. The acne quality of life scores, stress scores, and acne severity scores for the group of panelists described in Example A were calculated as described in Example A . In order to establish subsets of the panelist population, each individual's scores were classified as high (greater than the mean) or low (less than the mean).

In order to understand how stress and acne quality of life relate to severity, a count was taken to determine how many individuals had high vs. low, acne severity vs. stress vs. acne quality of life. A summary of this quantification is presented in Table B1.

**Table B1: Individual Acne Severity Score vs. AQOLI Score vs. PSS Score Classification Combinations**

| **Group** | **Level of Acne Quality of Life (AQOLI Score)** | **Level of Stress (PSS Score)** | **Level of Acne Severity (Severity Score)** | **Number of Participants** |
|---|---|---|---|---|
| 1 | High | High | High | 83 |
| 2 | High | High | Low | 43 |
| 3 | High | Low | High | 51 |
| 4 | High | Low | Low | 30 |
| 5 | Low | High | High | 35 |
| 6 | Low | High | Low | 85 |
| 7 | Low | Low | High | 35 |
| 8 | Low | Low | Low | 119 |

As seen in Table B1, combining both stress scores and acne quality of life scores with acne severity scores further segments the population indicating that acne patients not only differ in the severity of their acne and its impact on their quality of life, but also have varied levels of stress. By combining a stress assessment with our severity plus acne quality of life assessment combination, this example illustrates the further utility of our invention in better characterizing acne patients before determining treatment.

In summary, by utilizing a combination of an acne severity measure and an acne quality of life measure, Example A illustrates that our invention is a better method of assessing acne patients than the current practice of using severity assessment alone. As shown in Example B, further expanding the proposed combination of scores by using both a stress score and an acne quality of life score with an acne severity score enhances the method as even more segments of acne patients emerge. Each segment may need to be addressed differently in terms of treatments in order to ensure the best outcomes.

### Example C.: Subsets established by an acne quality of life measure (AQOLl ) + an acne severity measure (Avg. Number of Pimples) + a stress measure (TICS-2)

This example further illustrates the utility of an acne profile that includes acne quality of life, acne severity, and stress information. The example follows the same pattern as Example B, yet shows the use of different stress and acne severity measures and represents a different test population.

A group of 17 panelists with self-perceived acne participated in a six-week study to assess the relationship between various measures of skin condition, quality of life and stress. To assess acne severity over the course of the study, the group was given a diary and asked to record observed number of pimples on a daily basis. To assess stress over the course of the study, the group completed the Trier Inventory of Chronic Stress, version 2 (TICS-2) once a week. The TICS-2 is a 62-item psychometric tool that is used to assess the amount of chronic stress in an individual's life. The TICS-2 can be broken into 10 subscales in order to examine chronic stress factors in more depth. For the purposes of simplification, this example will focus on the total TICS-2 score.

To assess the impact of acne on their quality of life, the group completed the Acne Quality of Life Index (AQOLI) once a week throughout the study. A lower AQOLI indicates a more positive quality of life, i.e. a lower negative impact on quality of life due to acne. Thus, a relatively high AQOLI score indicates a relatively high impact of the individual's acne on his or her quality of life. Averages of the six weeks of TICS-2 scores, AQOLI scores, and Number of Pimples records were calculated for each individual. In order to establish subsets of the panelist population, each individual's average scores were classified as high (greater than the mean) or low (less than the mean). The average individual scores, group means and individual classifications are summarized in Table C1.

**Table C1: Individual Average Number of Pimples, TICS-2 Scores and AQOLI Scores (averaged over 6 weeks)**

| **Panelist #** | **Number of Pimples** | | **TICS-2 Score** | | **AQOLI Score** | |
|---|---|---|---|---|---|---|
| | **Mean** | **Class** | **Mean** | **Class** | **Mean** | **Class** |
| **1** | 0.58 | LOW | 0.44 | HIGH | 6 | LOW |
| **2** | 0.18 | LOW | 0.29 | LOW | 23 | HIGH |
| **3** | 1.92 | HIGH | 0.51 | HIGH | 39 | HIGH |
| **4** | 0.08 | LOW | 0.27 | LOW | 0 | LOW |
| **5** | 0.50 | LOW | 0.28 | LOW | 51 | HIGH |
| **6** | 0.21 | LOW | 0.35 | HIGH | 4 | LOW |
| **7** | 1.50 | HIGH | 0.41 | HIGH | 29 | HIGH |
| **8** | 0.66 | LOW | 0.26 | LOW | 11 | LOW |
| **9** | 1.26 | HIGH | 0.32 | LOW | 8 | LOW |
| **10** | 1.66 | HIGH | 0.41 | HIGH | 7 | LOW |
| **11** | 2.00 | HIGH | 0.37 | HIGH | 1 | LOW |
| **12** | 0.92 | HIGH | 0.51 | HIGH | 14 | HIGH |
| **13** | 0.00 | LOW | 0.44 | HIGH | 1 | LOW |
| **14** | 0.58 | LOW | 0.16 | LOW | 3 | LOW |
| **15** | 0.10 | LOW | 0.56 | HIGH | 37 | HIGH |
| **16** | 1.50 | HIGH | 0.22 | LOW | 4 | LOW |
| **17** | 0.06 | LOW | 0.09 | LOW | 0 | LOW |
| **Group Mean** | **0.81** | | **0.35** | | **14.00** | |

In order to understand how acne severity relates to acne quality of life, a count was taken to determine how many individuals had high vs. low, acne severity vs. acne quality of life. A summary of this quantification is presented in Table C2.

**Table C2: Individual Number of Pimples vs. AQOLI Score Classification Combinations**

| **Group** | **Level of Acne Severity (Number of Pimples)** | **Level of Acne Quality of Life (AQOLI Score)** | **Number of Participants** |
|---|---|---|---|
| 1 | High | Low | 4 |
| 2 | High | High | 3 |
| 3 | Low | Low | 7 |
| 4 | Low | High | 3 |

As seen in Table C2, there are individuals in each possible subgroup. As observed in Examples A & B, this is surprising because the common belief is that the level of acne impact on quality of life would be directly related to the severity of acne. Groups 1 and 4 illustrate that there are individuals who have high acne severity but low impact of acne on quality of life as well as individuals who have low acne severity by high impact of acne on quality of life. Without the use of a combination of acne severity and acne quality of life measures, the differences in the acne population would not be clear. The use of the combination would allow one to select a treatment customized to the individual's needs in terms of both acne severity and acne quality of life. Current dermatological practice utilizes only severity as its measure of a person's or population's acne. As shown here, the two populations identified by severity level greatly differ in terms of the impact that acne has on their quality of life. Thus, treatment based entirely on severity may not be as effective as it does not address the differences in acne's impact on quality of life.

The TICS-2 data from the same group of panelists illustrates the usefulness of adding a stress measure to the recommended combination of an acne severity measure and an acne quality of life measure. The TICS-2 average scores and high vs. low classifications are shown in Table C1. A count was taken to determine the number of individuals having the possible combinations of high vs. low, stress, acne severity, and acne quality of life. Table C3 summarizes the count.

**Table C3: Individual Number of Pimples vs. AQOLI Score vs. TICS-2 Score Classification Combinations**

| **Group** | **Level of Acne Severity (Number of Pimples)** | **Level of Acne Quality of Life (AQOLI Score)** | **Level of Stress (TICS-2)** | **Number of Participants** |
|---|---|---|---|---|
| 1 | High | High | Low | 0 |
| 2 | Low | High | Low | 2 |
| 3 | High | Low | Low | 2 |
| 4 | Low | Low | Low | 4 |
| 5 | High | High | High | 3 |
| 6 | Low | High | High | 1 |
| 7 | High | Low | High | 2 |
| 8 | Low | Low | High | 3 |

As seen in Table C3, adding the stress level further segments the population indicating that acne patients not only differ in the severity of acne and its impact on their quality of life, but also have varied levels of stress. The relationship between stress and acne is well established in the art, and thus it is important to investigate stress level in determining an appropriate acne treatment. By combining a stress assessment with our severity plus acne quality of life assessment combination, this example illustrates the further utility of our invention in better characterizing acne patients before determining treatment.

In summary, by utilizing a combination of an acne severity measure and an acne quality of life measure, this example illustrates that our invention is a better method of assessing acne patients than the current practice of using severity alone. Further expanding the proposed combination of measures by adding a stress measure enhances the method as even more segments of acne patients emerge. Each segment may need to be addressed differently in terms of treatments in order to ensure the best outcomes.

### Working Example D. Using The Acne Profile

In another embodiment of the invention, an individual concerned about their acne or skin is asked to complete the acne profile. Information is obtained from the individual about his or her acne quality of life. A preferred method of collecting the quality of life information is via administration of the AQOLI. The individual's total score on the Acne Quality of Life Index is calculated by summing the numerical equivalents of their responses and then subtracting this number from the total maximum score to yield the final score. Thus, the higher the final AQOLI score, the greater the negative impact of acne on this individual's quality of life. This total score comprises the first piece of information in the acne profile. Information is also obtained from the individual about the severity of his or her acne condition. For example, acne severity information could be obtained by counting the individual's number of acne lesions, thus establishing a number of acne lesions score. The individual's name or other identification information plus his or her acne quality of life and acne severity information is typed into the computer either by the individual himself or by a consultant upon obtaining the customer's information. The individual's AQOLI total score is compared to the average AQOLI score for a comparable population. Similarly, the individual's number of acne lesions score is compared to the mean number of lesions score for a comparable population to determine if the individual's number of acne lesions score is higher or lower than the population's mean. A preferred method of establishing the mean score or number of lesions for a comparable population is to take the average of scores or numbers of lesions for a population within the same (plus or minus 10 years) age range as the individual. If the individual's number of lesions score is higher than the mean number of lesions score, his acne severity will be classified as "HIGH". If the individual's number of lesions score is lower than the mean number of lesions score, his acne severity score will be classified as "LOW". Similarly, if the individual's AQOLI score is higher than the mean AQOLI score, his acne quality of life will be classified as "HIGH". If the individual's AQOLI score is lower than the mean AQOLI score, his quality of life will be classified as "LOW". In one embodiment, the information is formatted into an Acne Profile form such as that shown in Table D1.

**Table D1: Acne Profile Form**

| **ACNE PROFILE** | |
|---|---|
| **Name:** John Doe | **Date of Birth:** 01/05/82 |
| **Acne Quality of Life (AQOLI)** | HIGH |
| **Acne Severity (# of lesions)** | LOW |

The Acne Profile is then printed or emailed in order to share it with the individual, the individual's doctor or a skin care consultant.

### Working Example E: Selecting an Acne Treatment/Prevention Program

The acne profile according to the invention can be used to select a personalized acne treatment and/or prevention program for the individual being profiled. If the individual's acne quality of life score is classified as "LOW", the acne program includes a recommendation to maintain or lower the low negative impact of acne on the individual's quality of life. If the individual's quality of life score is classified as "HIGH", the acne program includes a recommendation to lower the high negative impact of acne on the individual's quality of life. If the individual's acne severity score is classified as "HIGH", the acne program includes recommendations to improve the individual's acne severity. If the individual's acne severity score is classified as "LOW", the acne program includes recommendations to maintain or further decrease the low severity of the individual's acne.

An example of the combined acne profile & program is illustrated in Table E1. In this example, the individual's acne quality of life is classified as "HIGH", and the individual's acne severity is classified as "LOW". To address the "HIGH" acne quality of life score, i.e. high impact of skin on the individual's quality of life, the acne program includes a recommendation to follow a daily sensory regimen. One example of a sensory regimen is taking a warm, relaxing bath while listening to relaxing music. To address the individual's "LOW" acne severity, the individual is given an acne prevention program that includes a mild cleanser to be used twice daily. While the low acne severity does not suggest the need for any type of acne treatment, a gentle product may be recommended in order to maintain the individual's low severity, to further decrease the severity, and/or to prevent future acne problems. Additionally, if acne quality of life is "HIGH", there is recognition that the skin condition is bothersome to the individual regardless of the severity. In this case, giving the individual a prevention regimen may be psychologically beneficial to the individual, as he will feel better about himself for making the effort to use the product to improve his skin condition.

**Table E1: Personalized Acne Profile & Prevention Program**

| **ACNE CONDITION PROFILE & PREVENTION PROGRAM** | | |
|---|---|---|
| **Name:** John Doe | **Date of Birth:** 01/05/82 | |
| **Acne Quality of Life (AQOLI)** | HIGH | **Recommendation**: Biweekly sensory regimen -10 min. warm, relaxing bath while listening to relaxing music |
| **Acne Severity (# of lesions)** | LOW | **Recommendation:** Use a mild cleanser to wash face two times per day |

The personalized Acne Profile & Prevention Program may be provided to the individual in a counseling session where a skin care consultant sits with the customer in a private or semi-private counseling area, which is a private or semi-private portion of the retail location. The consultant shows the customer his acne program and reviews the individual's acne program with him. Other options for delivery of the personalized acne profile & prevention program to the individual include the internet, email, phone or mail.

After he has implemented the recommendations for a time, the customer's acne severity and acne quality of life information could be collected again to establish an after-treatment acne profile. The customer's baseline acne profile would be compared to the after-treatment acne profile to determine the efficacy of the recommended acne treatment or prevention program. If the comparison indicates a satisfactory level of improvement, the customer would be instructed to continue with the original acne treatment or prevention program. If the comparison indicates an unsatisfactory level of improvement, the acne treatment or prevention program would be altered in a way to hopefully enhance its efficacy. For example, the program described in Table E1 could be altered by adding an acne treatment mask to the recommendations or by changing the products used in the sensory regimen. The process of getting an after-treatment acne profile and comparing it to one or more previous acne profiles could be continued repeatedly until an acceptable prevention or treatment program is found.

### Working Example F: Assessing the Efficacy of an Acne Treatment / Prevention Program

The acne profile according to the invention could also be used for assessing the efficacy of an acne treatment or prevention program. This application of the acne profiling method would be particularly useful in acne treatment or prevention product development when researchers need to determine which of two or more proposed acne treatment or prevention programs is most efficacious.

In this example, a researcher wants to determine the comparative efficacy of two acne treatment programs. The researcher would start by recruiting a group of acne study subjects. The study subjects would be divided into two groups - one for each acne treatment program to be tested. In this example, twenty study subjects are recruited. Ten study subjects are assigned to Group A and are thus given Treatment Program A. The other ten study subjects are assigned to Group B and thus given Treatment Program B. Before starting their respective treatment programs, the two groups of acne study subjects would first be profiled according to the method of the invention in order to establish baseline acne profile data sets. Acne quality of life and acne severity information would be obtained from each study subject. The subjects' acne quality of life scores and acne severity scores would be calculated resulting in an acne profile of each study subject. The acne profiles of the two groups of study subjects would then be combined resulting in a baseline data set of acne profiles for Group A and a baseline data set of acne profiles for Group B. The study subjects in both groups would be instructed to follow their assigned treatment program for a given amount of time, such as 8 weeks. Table F1 summarizes the subject groups and treatment program assignments for this example.

**Table F1: Summary of Acne Subject Groups & Treatment Program Assignments**

| | **Group A** | **Group B** |
|---|---|---|
| **Number of acne subjects** | 10 | 10 |
| **Treatment program assignment** | Treatment Program A (mild cleanser 2x per day) | Treatment Program B (mild cleanser 2x per day plus a daily warm bath with relaxing fragrance) |
| **Duration of assigned treatment program:** | 8 weeks | 8 weeks |

Following the assigned 8 weeks of the treatment programs, the study subjects in Groups A and B would be profiled again according to the method of the invention, resulting in after-treatment acne profiles of each study subject. The acne profiles of the subjects in Groups A and B would be compiled resulting in an after-treatment acne profile data set for Group A and an after-treatment acne profile data set for Group B.

A comparison could then be made between the baseline versus after-treatment acne profile data sets for each group. The comparisons would allow the researcher to see which of the two Treatment Programs, A or B, was most effective in improving the groups' acne profiles. For example, the researcher might observe that Treatment Program B reduced Group B's mean acne quality of life score more than Treatment Program A reduced Group A's mean acne quality of life score. A summary of results that could be obtained from a study like this is shown in Table F2.

**Table F2: Comparison of the Efficacy of Treatment Program A vs. B**

| **Acne Profile Comparison** | **Group A (Treatment Program A, 8 weeks)** | **Group B (Treatment Program B, 8 weeks)** |
|---|---|---|
| **% Change in Acne Quality of Life Score** | - 20% | -35% |
| **% Change in Acne Severity Score** | -15% | -15% |

As seen in Table F1, in this example, Group B's acne profiles improved more than Group A's in that Group B experienced a greater decrease in acne quality of life score. Thus, the researcher would be able to determine that Treatment Program B is more efficacious in improving acne conditions than Treatment Program A. The researcher may then decide to move forward with further development of Treatment Program B.

## Claims

1. A method of providing an acne treatment or prevention program to an individual said method comprising:
a. profiling the acne condition of said individual wherein said profiling comprises an acne quality of life score determined by obtaining and analyzing acne quality of life information from said individual, and optionally:
i. an acne severity score determined by obtaining and analyzing acne severity information from said individual;
ii. a stress score determined by obtaining and analyzing stress information from said individual, or;
iii. combinations thereof; and
b. recommending an acne treatment or prevention program.

2. The method of claim 1, wherein the acne quality of life information comprises information from at least one of the following dimensions:
a. psychological functioning;
b. social functioning;
c. mood;
d. relationship functioning;
e. burden of complying with current or proposed acne treatment program;
f. satisfaction with previously or currently used acne treatment programs; and
g. combinations thereof.

3. The profile of claim 2, wherein said acne quality of life information comprises information from at least one of the following dimensions:
a. psychological functioning;
b. social functioning;
c. mood; and
d. combinations thereof.

4. The method of claim 2, wherein the mood dimension comprises information obtained by asking the degree to which the individual's acne has made the individual feel at least one of the following:
a. happy;
b. sad;
c. angry;
d. uneasy;
e. frustrated;
f. mad;
g. uncomfortable;
h. ashamed;
i. stressed out;
j. anxious;
k. nervous;
l. down on himself;
m. touchy; and
n. upset.

5. The method of claim 2, wherein the psychological functioning dimension comprises information obtained by asking the degree to which the individual's acne has made the individual feel at least one of the following:
a. unhappy with his appearance;
b. less self-confident;
c. discouraged;
d. humiliated;
e. hopeless about his skin;
f. ugly;
g. self-pitying;
h. alone; and
i. like he can't be himself.

6. The method of claim 2, wherein the social functioning dimension comprises information obtained by asking the degree to which the individual's acne has made the individual feel at least one of the following:
a. uncomfortable around others;
b. self-conscious around others;
c. like he wants to avoid other people;
d. embarassed;
e. unattractive to others;
f. stared at; and
g. afraid of encountering people.

7. The method of claim 2, wherein the acne quality of life information is obtained using an acne quality of life psychometric tool.

8. The method of claim 7, wherein the acne quality of life psychometric tool is the AQOLI.

9. The method of claim 1, wherein said acne severity information comprises at least one of the following:
a. number of pimples;
b. severity of the pimples;
c. degree of dryness;
d. degree of oiliness;
e. degree of redness;
f. number of scars;
g. degree of skin roughness;
h. amount of skin pus;
i. number of blackheads;
j. number of whiteheads;
k. degree of pigmentation; and
l. combinations thereof.

10. The method of claim 9, wherein said acne severity information is obtained from the group consisting of:
a. dermatological grading;
b. skin image analysis;
c. trained self-assessment;
d. untrained self-assessment;
e. visual assessment by an untrained person other than the individual;
f. visual assessment by a trained person other than the individual;
g. assessment of the individual's image by an untrained person;
h. assessment of the individual's image by a trained person; and
i. a combination thereof.

11. The method of claim 1 wherein said stress information is obtained from the group consisting of
a. stress psychometric tool;
b. stress biometric tool; or
c. a combination thereof.

12. The method of claim 11 wherein said stress psychometric tool is selected from the group consisting of:
a. Trier Inventory of Chronic Stress, version 1;
b. Trier Inventory of Chronic Stress, version 2;
c. Perceived Stress Scale;
d. State-Trait Anxiety Inventory; and
e. combinations thereof.

13. The method of claim 12, wherein said stress psychometric tool is the Trier Inventory of Chronic Stress, version 2.

14. The method of claim 11 wherein said stress biometric tool is selected from the group consisting of:
a. salivary cortisol assessment;
b. heart rate variability assessment;
c. DRS assessment;
d. blood pressure;
e. skin temperature;
f. skin conductance;
g. heart rate; and
h. a combination thereof.

15. The method of claim 1, wherein said acne treatment or prevention program comprises elements that address acne quality of life, and optionally:
i. elements that address acne severity;
ii. elements that address stress, or;
iii. combinations thereof.

16. The method of claim 1, wherein if the acne quality of life score for said individual is higher than a benchmark acne quality of life score, than an acne treatment or prevention program that includes elements that address acne quality of life is recommended.

17. The method of claim 15, wherein said elements that address acne quality of life comprise a recommendation to: seek psychological, emotional, and social counseling; start an exercise program; practice behavioral modification techniques; practice relaxation techniques; practice breathing techniques; improve sleep patterns; modify current acne treatment program so that it is less burdensome or more satisfying to the individual; modify current acne prevention program so that it is less burdensome or more satisfying to the individual and combinations thereof.

18. The method of claim 15, wherein said elements that address acne quality of life comprise a recommendation to practice a sensory regimen.

19. The method of claim 1, wherein if the acne severity score for said individual is higher than a benchmark acne severity score, then an acne treatment or prevention program that includes elements that address acne severity is recommended.

20. The method of claim 1, wherein if the stress score for said individual is higher than a benchmark stress score, then an acne treatment or prevention program that includes elements that address stress is recommended.

21. The method of claim 15, wherein said elements that address acne severity include the administration of an anti-acne composition.

22. A method according to claim 21, wherein the anti-acne composition is administered orally or topically.

23. A method according to claim 22, wherein the anti-acne composition is administered topically and comprises an anti-acne agent selected from salicylic acid, sulfur, lactic acid, glycolic acid, pyruvic acid, urea, resorcinol, N-acetylcysteine, retinoic acid, benzoyl peroxide, octopirox, triclosan, azelaic acid, phenoxyethanol, phenoxypropanol, flavinoids, derivatives thereof, and mixtures thereof.

24. A method according to claim 22, wherein the anti-acne composition comprises an anti-acne agent selected from isotretinoin and tretinoin, adapalene, tazarotene, azelaic acid, minocycline, doxycycline, erythromycin and clindamycin and mixtures thereof.

25. A method of assessing the efficacy of an acne treatment or prevention program, said method comprising:
a. profiling the baseline acne condition of an individual prior to the administration of said acne treatment or prevention program wherein said profiling comprises an acne quality of life score determined by obtaining and analyzing acne quality of life information from said individual, and optionally:
i. an acne severity score determined by obtaining and analyzing acne severity information from said individual;
ii. a stress score determined by obtaining and analyzing stress information from said individual, or;
iii. combinations thereof;
b. recommending an acne treatment or prevention program wherein said acne treatment or prevention program comprises elements that address acne quality of life, and optionally:
i. elements that address acne severity;
ii. elements that address stress, or;
iii. combinations thereof.
c. profiling the after-treatment acne condition of said individual after administering said treatment or prevention program wherein said profiling comprises an acne quality of life score determined by obtaining and analyzing acne quality of life information from said individual, and optionally:
i. an acne severity score determined by obtaining and analyzing acne severity information from said individual;
ii. a stress score determined by obtaining and analyzing stress information from said individual, or;
iii. combinations thereof;
d. comparing said profiled baseline acne condition to said profiled after-treatment acne condition to determine the efficacy of said acne treatment or prevention program.

26. A method of selecting an acne treatment or prevention program, said method comprising:
a. assessing the efficacy of two or more proposed acne treatment or prevention programs according to the method of claim 25;
b. comparing the efficacy of the two or more proposed acne treatment or prevention programs; and
c. selecting the most efficacious acne treatment or prevention program for further development.

27. A method of providing an acne treatment or prevention program to an individual said method comprising:
a. profiling the acne condition of said individual wherein said profiling comprises an acne quality of life score determined by obtaining and analyzing acne quality of life information from said individual using the AQOLI, and optionally:
i. an acne severity score determined by obtaining and analyzing acne severity information from said individual;
ii. a stress score determined by obtaining and analyzing stress information from said individual, or;
iii. combinations thereof; and
b. recommending an acne treatment or prevention program wherein said acne treatment or prevention program comprises elements that address acne quality of life, and optionally:
i. elements that address acne severity;
ii. elements that address stress, or;
iii. combinations thereof.
